# EUROPEAN PATENT SPECIFICATION

(11) **EP 2 624 842 B1**
(45) Date of publication and mention of the grant of the patent: **15.03.2017**
(21) Application number: 11831673.6
(22) Date of filing: 07.10.2011
(51) Int. Cl.: A61K 31/728, A61K 38/39, A61K 38/18, A61P 7/06

(54) **USE OF HYALURONAN FOR PROMOTING ANGIOGENESIS**
VERWENDUNG VON HYALURONAN ZUR FÖRDERUNG EINER ANGIOGENESE
UTILISATION DE HYALURONANE POUR STIMULER L'ANGIOGENÈSE

(30) Priority: 07.10.2010 US 390789 P
(43) Date of publication of application: 14.08.2013
(73) Proprietor: National Cheng Kung University, Tainan, (TW)
(72) Inventor: HUANG, Lynn, L., H., 70101 (TW)
(74) Representative: Becker Kurig Straus
(86) International application number: PCT/US2011/055288
(87) International publication number: WO 2012/048214

(56) References cited:
- US-A1- 2003 157 161
- US-A1- 2010 144 635
- ERIN L L. PARDUE: "Role of hyaluronan in angiogenesis and its utility to angiogenic tissue engineering", ORGANOGENESIS, vol. 4, no. 4, 1 October 2008 (2008-10-01) , pages 203-214, XP055113419,
- GAO F ET AL: "Hyaluronan oligosaccharides promote excisional wound healing through enhanced angiogenesis", MATRIX BIOLOGY, ELSEVIER, NL, vol. 29, no. 2, 1 March 2010 (2010-03-01), pages 107-116, XP026911423, ISSN: 0945-053X, DOI: 10.1016/J.MATBIO.2009.11.002 [retrieved on 2009-11-12]

## Description

Hyaluronan, also known as hyaluronic acid or hyaluronate, is an anionic, non-sulfated glycosaminoglycan found in various animal tissues (e.g., skin, cartilage, and the vitreous humour) and in microbial extracellular capsules. In nature, it is synthesized by hyaluronan synthases and has a molecular weight of 5 to 20,000 kDa.

Hyaluronan has various medical applications. As a naturally occurring polymer, it is commonly used for preparing biomaterial scaffold in tissue engineering. Further, hyaluronan is applied to osteoarthritis patients to supplement the viscosity of joint fluids, thereby enhancing lubrication of joints and consequently reducing pain. Hyaluronan biomedical products can also serve as lubricants in eye surgery.

US 2003/157161 A1 is concerned with a composition and method for treating inflammatory conditions using proteins or polysaccharides containing anti-microtubule agents. The polysaccharide of the composition may also contain hyaluronan or a derivate thereof with a molecular weight greater than 12 kDa, or 50 kDa to 900 kDa up to 1600 kDa.

US 2010/144635 A1 refers to a solution introduced to an infarct zone of ischemic heart. The composition comprises hyaluronic acid, collagen growth factors and gelatin.

Erin L L. Pardue: "Role of hyaluronan is angiogenesis and its utility to agiogenic tissue engineering" discloses the results of an in-vitro endothelial cells (EC's) culture that show that hyaluronan oligomers promote angiogenesis.

Gao F et al: "Hyaluronan oligosaccharides promote excisional wound healing through enhanced agiogenesis" refers to hyaluronan oligosaccharides and the promotion of wound healing through enhanced angiogenesis.

The present invention is based on an unexpected discovery that hyaluronan, particularly hyaluronan of high molecular weight, is effective in promoting angiogenesis, thereby facilitating wound recovery.

Accordingly, this invention features a composition for use in promoting angiogenesis by administering to a subject at a site adjacent to a wound caused by ischemia. The composition containing hyaluronan (e.g., long-chain hyaluronan having a molecular weight at least 12 kDa) at a concentration effective for stimulating local angiogenesis, e.g., 0.02 to 50 mg/ml or 2 to 20 mg/ml. Hyaluronan used has a molecular weight of 50 kDa to 2,000 kDa (e.g., 70 kDa to 1,500 kDa, 200 kDa to 1,500 kDa, 500 kDa to 1,500 kDa, or 700 kDa to 1,500 kDa). The subject can be a human patient having a wound caused by, ischemia. To promote angiogenesis, the hyaluronan-containing composition can be administered via, e.g., intramuscular injection, at a site on or near the wound for stimulating local angiogenesis, thereby promoting wound recovery. In one example, the hyaluronan-containing composition is free of growth factors and cells. In another example, it further contains a growth factor, a cell, or a combination thereof. Any of the hyaluronan-containing composition can further contain collagen or gelatin.

The details of one or more embodiments of the invention are set forth in the description below. Other features or advantages of the present invention will be apparent from the following example and also from the appended claims.

Described herein is a a composition for use in treating wound caused by ischemia promoting angiogenesis nearby a wound caused by ischemia, using hyaluronan, either taken alone or in combination with one or more other therapeutic agents, e.g., collagen, gelatin, growth factor, and stem cell.

The term "hyaluronan" refers to a naturally-occurring glycosaminoglycan polymer including repeated disaccharide units of N-acetylglucosamine and D-glucuronic acid, and its derivatives. Naturally-occurring hyaluronan, having the formula of (C₁₄H₂₁NO₁₁)ₙ, can be obtained via conventional methods. In one example, it is isolated from its natural sources, e.g., capsules of Streptococci, rooster comb, cartilage, synovial joints fluid, umbilical cord, skin tissue and vitreous of eyes, via conventional methods. See, e.g., Guillermo Lago et al. Carbohydrate Polymers 62(4): 321-326, 2005; and Ichika Amagai et al. Fisheries Science 75(3): 805-810, 2009. In another example, it can be synthesized in a genetically engineered microorganism suitable for producing hyaluronan. Typically, hyaluronan thus obtained is heterogenous, i.e., including molecules with different lengths and therefore different molecular weights. Hyaluronan molecules within a particular range of molecular weights can be obtained using a filter with a particular molecular weight cutoff or by gel filtration. Alternatively, hyaluronan can be purchased from a commercial vendor, e.g., Genzyme Corporation, Lifecore Biomedical, LLC and Hyaluron Contract Manufacturing.

Derivatives of naturally-occurring hyaluronan include, but are not limited to, hyaluronan esters, adipic dihydrazide -modified hyaluronan, hyaluronan amide products, crosslinked hyaluronic acid, hemiesters of succinic acid or heavy metal salts thereof hyaluronic acid, partial or total esters of hyaluronic acid, sulphated hyaluronic acid, N-sulphated hyaluronic acid, and amines or diamines modified hyaluronic acid. They can be obtained by chemically modifying one or more of its functional groups (e.g., carboxylic acid group, hydroxyl group, reducing end group, N-acetyl group). A carboxyl group can be modified via esterification or reactions mediated by carbodiimid and bishydrazide. Modifications of hydroxyl groups include, but are not limited to, sulfation, esterification, isourea coupling, cyanogen bromide activation, and periodate oxidation. A reducing end group can be modified by reductive amination. It also can be linked to a phospholipid, a dye (e.g., a fluorophore or chromophore), or an agent suitable for preparation of affinity matrices. Derivatives of naturally-occurring hyaluronan can also be obtained by crosslinking, using a crosslinking agent (e.g., bisepoxide, divinylsulfone, biscarbodiimide, small homobifunctional linker, formaldehyde, cyclohexyl isocyanide, and lysine ethyl ester, metal cation, hydrazide, or a mixture thereof) or via internal esterification, photocross-linking, or surface plasma treatment.

The hyaluronan described above can be mixed with a pharmaceutically acceptable carrier to form a pharmaceutical composition. A pharmaceutically acceptable carrier is compatible with the active ingredient of the formulation (and preferably, capable of stabilizing it) and not deleterious to the subject to be treated. For example, solubilizing agents such as cyclodextrins, which form specific, more soluble complexes with hyaluronan, or one or more solubilizing agents, can be utilized as pharmaceutical excipients for delivery of hyaluronan. Examples of other carriers include colloidal silicon dioxide, magnesium stearate, cellulose, sodium lauryl sulfate, and D&C Yellow # 10.

The hyaluronan-containing pharmaceutical composition described above can further contain collagen or gelatin (derived from denatured collagen such as boiling). Any of the naturally-occurring collagens or their functional variants can be used for preparing this composition. At the present time, at least 20 genetically distinct species of collagens have been discovered. Collagen can be easily isolated and purified from collagen-rich tissues such as skin, tendon, ligament, and bone of humans and animals. Methods for isolating and purifying collagen are well known in the art. See, e.g., US Patent 5,512,291; US Patent Publication 20040138695; Methods in Enzymology, vol. 82, pp. 33-64, 1982; The Preparation of Highly Purified Insoluble Collagen, Oneson, I., et al., Am. Leather Chemists Assoc., Vol. LXV, pp. 440-450, 1970; U.S. Pat. No. 6,090,996). Collagen can also be prepared by recombinant technology, such as those described by Advanced Tissue Sciences (La Jolla, Calif.) or purchased from various venders (e.g., Fibrogen; South San Francisco, Calif). One example follows. Bovine deep flexor tendons, with fat and fascia removed, are washed with water, frozen, and sliced into 0.5 mm slices with a meat slicer. A suitable amount of the sliced tendons is first extracted with 50 ml of water at room temperature for 24 hours. The water-soluble fraction is discarded and the spliced tendons are then extracted with an acidic solution (e.g., 0.2 N HCl) at a suitable temperature (e.g., room temperature) for a suitable period of time (e.g., 12-24 hours). The HCl solution is discarded; the tendons rinsed with water to remove the residual acid. The rinsed tendons are then extracted with a basic solution (e.g.,0.75 M NaOH) at a suitable temperature (e.g., room temperature) for a suitable period of time (e.g., 12-24 hours). After discarding the basic solution, the sliced tendons are neutralized with an acidic solution (e.g., 0.1 N HCl) to a pH of 4-7 (e.g. 5) followed by repetitive washes with water to remove the residual base in the tendons. The tendons are then defatted with an alcohol (e.g., isopropanol) for a sufficient period (e.g., 16 hours) at room temperature. The extractant is decanted and the tendons are further extracted with an alcohol (e.g., isopropanol) for a suitable period (e.g., 12-24 hours) at room temperature for form a collagen-containing solution, which can be dried under a clean hood. The collagen powder thus formed can be dispersed in an acidic solution (e.g., 0.5 M or 0.25 M acetic acid) in the presence of a proteolytic enzyme (e.g., trypsin or pepsin) and incubated at 4 °C for a suitable period. The mixture is then filtered through a 100 mesh stainless steel mesh filter and the solubilized collagen can be precipitated with a 5% NaCl solution. The precipitated collagen can be redissolved in the acidic solution described above and the solution thus formed can be filtered through a 100 mesh stainless steel mesh filter to eliminate nonsolubilized particles. The collagen solution is then dialyzed with distilled water to remove the acid.

Alternatively or in addition, the hyaluronan-containing pharmaceutical composition described above further contains a bioactive agent (e.g., peptide, polypeptide, oligosaccharide, polysaccharide, or small molecule) that promotes cell proliferation, angiogenesis, or wound healing. In one example, the bioactive agent is a growth factor, such as epidermal growth factor, fibroblast growth factor, vascular endothelial growth factor, connective tissue growth factor, platelet-derived growth factor, insulin-like growth factor, nerve growth factor, hepatocyte growth factor, colony-stimulating factors, stem cell factor, serotonin, and von Willebrand factor, transforming growth factor, keratinocyte growth factor, granulocyte colony-stimulating factor, granulocyte/macrophage colony stimulating factor, glial derived neurotrophic factor, ciliary neurotrophic factor, endothelial-monocyte activating polypeptide, epithelial neutrophil activating peptide, erythropoietin, bone morphogenetic proteins, brain-derived neurotrophic factor. In another example, the bioactive agent is a cytokine or chemokine, including, but are not limited to, IL-2, breast-expressed chemokine (e.g., BRAK), kidney-expressed chemokine (e.g., CXCL14). The bioactive agent can also be a cell differentiation factor, such as dexamethasone, sodium pyruvate, ascorbic acid-2-phosphate, proline, insuline, transferrin, selenous acid, linoleic acid, and bovine serum albumin, and TGF-ß3. In a preferred example, the differentiation factor is a compound that promotes chondrogenesis of mesenchymal stem cells (see those disclosed in US Patent 5,908,784), osteogenesis (e.g., dexamethasone, ascorbic acid, β-glycerol phosphate), adipogenesis (e.g., insulin, isobutyl-methyl xanthine, dexamethasone, indomethacin), cardiomyogenic differentiation (e.g., activin A, BMP-4), endothelial cell differentiation (e.g., EBM-2, dexamethasone, and VEGF), smooth muscle cell differentiation (e.g., PDGF-BB), neural induction (e.g., bFGF, EGF, and B27 supplement, DMSO, butylated hydroxyanisole, forskolin, valproic acid, KCl, K252a, and N2 supplement) and endodermal lineage differentiation( e.g., dexamethasone, HGF, and FGF-4). The bioactive agent can also be a Chinese herbal medicine or an active ingredient thereof.

To promote local angiogenesis, any of the pharmaceutical compositions mentioned above can be administered at one or more sites on or nearby a region of interest via, e.g., intramuscular injection or an implanted reservoir. A region of interest can be a wound caused by ischemia, heart infarct, diabetes, eye injury, ulcer, or chronic wounds, etc. Healing of such a wound normally requires angiogenesis, i.e., formation of new blood vessels, which can be detected by monitoring changes of blood flow at the region of interest. The concentration of hyaluronan in the composition can range from 0.02 mg/ml to 50 mg/ml (e.g., 2 mg/ml to 20 mg/ml, 2 mg/ml to 10 mg/ml, or 5 mg/ml to 10 mg/ml). It can be varied based on the molecular weight of the hyaluronan in the composition.

A sterile injectable composition, e.g., a sterile injectable aqueous or oleaginous suspension, can be formulated according to techniques known in the art using suitable dispersing or wetting agents (such as Tween 80) and suspending agents. The sterile injectable preparation can also be a sterile injectable solution or suspension in a non-toxic parenterally acceptable diluent or solvent, for example, as a solution in 1,3-butanediol. Among the acceptable vehicles and solvents that can be employed are mannitol, water, Ringer's solution and isotonic sodium chloride solution. In addition, sterile, fixed oils are conventionally employed as a solvent or suspending medium (e.g., synthetic mono- or diglycerides). Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions can also contain a long-chain alcohol diluent or dispersant, or carboxymethyl cellulose or similar dispersing agents. Other commonly used surfactants such as Tweens or Spans or other similar emulsifying agents or bioavailability enhancers which are commonly used in the manufacture of pharmaceutically acceptable solid, liquid, or other dosage forms can also be used for the purposes of formulation.

Without further elaboration, it is believed that one skilled in the art can, based on the above description, utilize the present invention to its fullest extent. The following specific embodiments are, therefore, to be construed as merely illustrative, and not limitative of the remainder of the disclosure in any way whatsoever. All publications cited herein are incorporated by reference.

### EXAMPLE 1: Effect of Hyaluronan in Promoting Angiogenesis in Ischemic Limbs in Diabetic Mice and Improving Ischemic Limb Recovery

Male C57BL/6J Narl (6-8 weeks old) mice were obtained from National Experimental Laboratory Animal Center (NLAC, Tainan, Taiwan). Experimental diabetes was induced in these mice by daily intra-peritoneal injection of Streptozotocin (STZ) in citrate buffer (50 mg/kg body weight) for 5 day. One week after the STZ injection, the mice developed significant hypoinsulinemia and severe hyperglycemia (serum glucose > 300mg/dl).

Hind limb ischemia was induced in both STZ-untreated (normal) and STZ-treated (diabetic) mice following the method described in Tang et al., Journal of vascular surgery 41:312-320 (2005) and Yan et al., Journal of vascular surgery 50:1412-1422 (2009). Briefly, mice were anesthetized using 2% isoflurane. Ketoprofen was used as an analgesic. Their left femoral arteries and associated vessel branches were isolated and ligated. The left femoral artery in each mouse was excised from the inguinal ligment to the bifurcation of the saphenous and popliteal arteries. After excision, hyaluronic acids having different molecular weights (i.e., 2 kD, 780 kD, 1500 kD, and 2000 kDa), obtained from Lifecore Biomedical, LLC., were injected into the left hind limbs intramuscularly at various concentrations (i.e., 0.5 mg/ml, 2 mg/ml, 5 mg/ml, and 10 mg/ml).

Blood flow in the hind limb of each mouse, indicating local angiogenesis, was measured as follows. A laser Doppler perfusion imager (Moor Instruments Ltd., Devon, United Kingdom) was used to estimate dermal blood flow in bilateral hind limbs. The mice were anesthetized with 1.5% isoflurane, their hind limb furs removed by depilatory cream. This study was conducted in a warm (37°C) and darkened room to minimize the effects of ambient light and temperature. The levels of blood flow obtained from ischemic hind limbs were normalized against those from nonischemic hind limbs. The results thus obtained were shown in Table 1 below:

**Table 1. Effect of hyaluronan in promoting blood flow in ischemic hind limbs of diabetic mice**

| **ROI %*** | PBS control | Hyaluronan Concentration (mg/ml) | | | |
|---|---|---|---|---|---|
| | | 0.5 mg/mL | 2 mg/mL | 5 mg/mL | 10 mg/mL |
| Hyaluronan M.W. | | | | | |
| 12k | - | | | 63.7 | 44.8 |
| 780k | - | 52.0 | 52.4 | 61.3 | |
| 1500k | - | | | 51.8 | 49.1 |
| 2000k | - | | 52.3 | 60.0 | |
| PBS control | 10.6 | - | - | - | - |

| | | | | | |
|---|---|---|---|---|---|
| * ROI refers to region of interest. The values of ROI% listed in this table were calculated as follows: (Blood flow level of a region of interest in an ischemic hind limb) / (Blood flow level of a region of interest in a non-ischemic hind limb) %. | | | | | |

As shown in Table 1, hyaluronan having any of the listed molecular weights promoted blood flow in ischemic hind limbs of diabetic mice as compared with blood flow in non-ischemic hind limbs. This result indicates that hyaluronan improved angiogenesis in ischemic hind limbs.

Physical examination was performed to assess both morphological and functional changes of hind limbs 2 and 4 weeks after ischemia was induced, following the scale table below:

**Table 2. The scale of recovery score for mice determined by gross observation**

| **Score** | **Status** | **Score** | **Status** |
|---|---|---|---|
| **0** | Amputation of leg | **8** | Amputation of one toe |
| **1** | Necrotic leg | **9** | Multiple necrotic toes |
| **2** | Amputation of foot | **10** | One necrotic toe |
| **3** | Necrotic foot | **11** | Tips of multiple toes blackened |
| **4** | Necrotic calcaneum | **12** | Tip of one toe blackened |
| **5** | Amputation of paw | **13** | Multiple claws blackened/broken off |
| **6** | Necrotic paw | **14** | One claw blackened/broken off |
| **7** | Amputation of toes | **15** | Normal |

Recovery scores for mice treated with hyaluronan were determined by gross observation.

As shown in Table 3 below, hyaluronan was effective in facilitating hind limb recovery from ischemia.

**Table 3. Effects of hyaluronan in ischemic hind limb recovery**

| Score | PBS control | Hyaluronan Concentration (mg/ml) | | | |
|---|---|---|---|---|---|
| | | 0.5 mg/mL | 2 mg/mL | 5 mg/mL | 10 mg/mL |
| Hyaluronan M.W. | | | | | |
| 12k | - | | | 13 | 15 |
| 780k | - | 14 | 15 | 15 | |
| 1500k | - | | | 15 | 12.5 |
| 2000k | - | | 13 | 13 | |
| PBS control | 3 | - | - | - | - |

In all of the studies described above, the contralateral hind limb served as an internal control in each mouse.

### EXAMPLE 2: Effect of Hyaluronan and Collagen in Promoting Angiogenesis in Ischemic Hind Limbs and Improving Ischemic Hind Limb Recovery in Diabetic Mice

Ischemia was induced in hind limbs of STZ-treated diabetic mice, following the method described in Example 1 above. 100 ul of a composition containing both hyaluronan (with different molecular weights as shown in Tables 4 and 5 below at 10 mg/ml) and collagen (10 mg/ml) was injected intramuscularly into the ischemic hind limbs. Blood flow and morphological/functional changes in the treated mice were examined at various time points, following the procedures also described in Example 1 above. As shown in Tables 4 and 5 below, the combination of hyaluronan and collagen also promoted blood flow in ischemic hind limbs and morphological/functional recovery of the wounded limbs.

**Table 4. Effects of hyaluronan and collagen on promoting blood flow in ischemic hind limbs of diabetic mice**

| | **ROI %*** | | | | | |
|---|---|---|---|---|---|---|
| | Day 0 | Day 3 | Week 1 | Week 2 | Week 3 | Week 4 |
| Hyaluronan M.W. | | | | | | |
| 4.3k | 8.9 | 10.0 | 11.3 | 32.4 | 28.6 | 58.7 |
| 12k | 7.6 | 10.9 | 20.2 | 49.8 | 32.7 | 51.7 |
| 130k | 8.2 | 12.0 | 27.7 | 38.4 | 42.8 | 55.0 |
| 1500k | 11.1 | 10.8 | 30.5 | 51.5 | 45.2 | 72.0 |
| 2590k | 7.9 | 5.7 | 11.9 | 25.1 | 29.8 | 29.2 |
| Collagen alone | 7.8 | 10.1 | 18.9 | 40.3 | 50.2 | 43.6 |

**Table 5. Effects of hyaluronan and collagen in ischemic hind limb recovery**

| | **Score** | |
|---|---|---|
| Hyaluronan M.W. | Week 2 | Week 4 |
| 4.3k | 10.0 | 9.0 |
| 12k | 12.8 | 13.5 |
| 130k | 13.5 | 14.0 |
| 1500k | 13.7 | 13.7 |
| 2590k | 5.0 | 5.0 |
| Collagen alone | 11.7 | 10.3 |

In table 5, physical examination was performed to assess both morphological and functional changes of hind limbs 2 and 4 weeks after ischemia was induced, following the scale in the table 2. Recovery scores for mice treated with hyaluronan were determined by gross observation.

## Claims

1. A composition for use in treating wound caused by ischemia by promoting angiogenesis, the composition comprising a hyaluronan or derivatives thereof with a molecular weight between 50 kDa and 2,000 kDa, wherein the concentration of the hyaluronan is effective for promoting angiogenesis, and wherein the composition is administered at or to a site adjacent to a wound caused by ischemia.

2. The composition for use according to claim 1, wherein the molecular weight of the hyaluronan or its derivatives is 200 kDa to 1,500 kDa.

3. The composition for use according to claim 2, wherein the molecular weight of the hyaluronan or its derivatives is 700 kDa to 1,500 kDa.

4. The composition for use according to claim 1, wherein the concentration of the hyaluronan or its derivatives is 0.02 to 50 mg/ml.

5. The composition for use according to claim 4, wherein the concentration of the hyaluronan or its derivatives is 2 to 20 mg/ml.

6. The composition for use according to claim 5, wherein the composition is administered intramuscularly.

7. The composition for use according to claim 1, wherein the composition further contains collagen, gelatin, growth factor, or a combination thereof.

8. The composition for use according to claim 1, wherein the composition is free of growth factor and cell.

9. The composition for use according to claim 8, wherein the composition further contains collagen.

10. The composition for use according to any one of claims 1 to 9, wherein the composition is administered to a human.

11. Use of hyaluronan or derivatives thereof with a molecular weight between 50 kDa and 2,000 kDa for the manufacture of a medicament for treating wound caused by ischemia by promoting angiogenesis, wherein the composition is administered at or to a site adjacent to a wound caused by ischemia.

12. The use according to claim 11, wherein the molecular weight of the hyaluronan or its derivatives is 200 kDa to 1,500 kDa, preferably 700 kDa to 1,500 kDa.

13. The use according to claim 11, wherein the concentration of the hyaluronan or its derivatives is 0.02 to 50 mg/ml.

14. The use according to claim 13, wherein the concentration of the hyaluronan or its derivatives is 2 to 20 mg/ml.

15. The use according to claim 14, wherein the composition is administered intramuscularly.

16. The use according to claim 11, wherein the composition further contains collagen, gelatin, growth factor, or a combination thereof.

17. The use according to claim 11, wherein the composition is free of growth factor and cell.

18. The use according to claim 17, wherein the composition further contains collagen.

19. The use according to any one of claims 11 to 18, wherein the composition is administered to a human.

## Patentansprüche

1. Zusammensetzung zur Verwendung zur Behandlung von Wunden, die durch Ischämie verursacht wurden, durch geförderte Angiogenese, wobei die Zusammensetzung umfasst:
eine Hyaluronsäure oder Derivate davon mit einem Molekulargewicht zwischen 50 kDa und 2.000 kDa, wobei die Konzentration der Hyaluronsäure wirksam ist, um die Angiogenese zu fördern, und wobei die Zusammensetzung bei oder an einer Stelle nahe der Wunde verabreicht wird, die durch Ischämie verursacht wurde.

2. Zusammensetzung zur Verwendung gemäß Anspruch 1, wobei das Molekulargewicht der Hyaluronsäure oder deren Derivaten von 200 kDa bis 1.500 kDa beträgt.

3. Zusammensetzung zur Verwendung gemäß Anspruch 2, wobei das Molekulargewicht der Hyaluronsäure oder deren Derivaten von 700 kDa bis 1.500 kDa beträgt.

4. Zusammensetzung zur Verwendung gemäß Anspruch 1, wobei die Konzentration der Hyaluronsäure oder deren Derivaten von 0,02 bis 50 mg/ml beträgt.

5. Zusammensetzung zur Verwendung gemäß Anspruch 4, wobei die Konzentration der Hyaluronsäure oder deren Derivaten von 2 bis 20 mg/ml beträgt.

6. Zusammensetzung zur Verwendung gemäß Anspruch 5, wobei die Zusammensetzung intramuskulär verabreicht wird.

7. Zusammensetzung zur Verwendung gemäß Anspruch 1, wobei die Zusammensetzung weiterhin Collagen, Gelatine, einen Wachstumsfaktor oder eine Kombination davon enthält.

8. Zusammensetzung zur Verwendung gemäß Anspruch 1, wobei die Zusammensetzung frei von Wachstumsfaktor und Zelle ist.

9. Zusammensetzung zur Verwendung gemäß Anspruch 8, wobei die Zusammensetzung weiterhin Collagen enthält.

10. Zusammensetzung zur Verwendung gemäß irgendeinem der Ansprüche 1 bis 9, wobei die Zusammensetzung einem Menschen verabreicht wird.

11. Verwendung einer Hyaluronsäure oder Derivaten davon mit einem Molekulargewicht zwischen 50 kDa und 2.000 kDa zur Herstellung eines Medikaments zur Behandlung von Wunden, die durch Ischämie verursacht wurden, durch Förderung von Angiogenese, wobei die Zusammensetzung bei oder an einer Stelle nahe der Wunde verabreicht wird, die durch Ischämie verursacht wurde.

12. Verwendung gemäß Anspruch 11, wobei das Molekulargewicht der Hyaluronsäure oder deren Derivaten von 200 kDa bis 1.500 kDa, bevorzugt von 700 kDa bis 1.500 kDa beträgt.

13. Verwendung gemäß Anspruch 11, wobei die Konzentration der Hyaluronsäure oder deren Derivaten von 0,02 bis 50 mg/ml beträgt.

14. Verwendung gemäß Anspruch 13, wobei die Konzentration der Hyaluronsäure oder deren Derivaten von 2 bis 20 mg/ml beträgt.

15. Verwendung gemäß Anspruch 14, wobei die Zusammensetzung intramuskulär verabreicht wird.

16. Verwendung gemäß Anspruch 11, wobei die Zusammensetzung weiterhin Collagen, Gelatine, einen Wachstumsfaktor oder eine Kombination davon enthält.

17. Verwendung gemäß Anspruch 11, wobei die Zusammensetzung frei von Wachstumsfaktor und Zelle ist.

18. Verwendung gemäß Anspruch 17, wobei die Zusammensetzung weiterhin Collagen enthält.

19. Verwendung gemäß irgendeinem der Ansprüche 11 bis 18, wobei die Zusammensetzung einem Menschen verabreicht wird.

## Revendications

1. Composition pour une utilisation dans le traitement d'une plaie ischémique en favorisant l'anglogenèse, la composition comprenant :
un hyaluronane ou des dérivés de celui-ci ayant un poids moléculaire compris entre 50 kDa et 2 000 kDa,
dans laquelle la concentration en hyaluronane est efficace pour favoriser l'angiogenèse, et
dans laquelle la composition est administrée au niveau ou à côté d'une plaie ischémique.

2. Composition pour une utilisation selon la revendication 1, dans laquelle le poids moléculaire de l'hyaluronane ou de ses dérivés est compris entre 200 kDa et 1 500 kDa.

3. Composition pour une utilisation selon la revendication 2, dans laquelle le poids moléculaire de l'hyaluronane ou de ses dérivés est compris entre 700 kDa et 1 500 kDa.

4. Composition pour une utilisation selon la revendication 1, dans laquelle la concentration en hyaluronane ou en ses dérivés est comprise entre 0,02 et 50 mg/ml.

5. Composition pour une utilisation selon la revendication 4, dans laquelle la concentration en hyaluronane ou en ses dérivés est comprise entre 2 et 20 mg/ml.

6. Composition pour une utilisation selon la revendication 5, laquelle composition est administrée par voie intramusculaire.

7. Composition pour une utilisation selon la revendication 1, laquelle composition contient en outre du collagène, de la gélatine, un facteur de croissance ou une combinaison de ceux-ci.

8. Composition pour une utilisation selon la revendication 1, laquelle composition est exempte de facteur de croissance et de cellule.

9. Composition pour une utilisation selon la revendication 8, laquelle composition contient en outre du collagène.

10. Composition pour une utilisation selon l'une quelconque des revendications 1 à 9, laquelle composition est administrée à un être humain.

11. Utilisation d'hyaluronane ou de dérivés de celui-ci ayant un poids moléculaire compris entre 50 kDa et 2 000 kDa pour la fabrication d'un médicament pour le traitement d'une plaie ischémique en favorisant l'angiogenèse,
dans laquelle la composition est administrée au niveau ou à côté d'une plaie ischémique.

12. Utilisation selon la revendication 11, dans laquelle le poids moléculaire de l'hyaluronane ou de ses dérivés est compris entre 200 kDa et 1 500 kDa, de préférence entre 700 kDa et 1 500 kDa.

13. Utilisation selon la revendication 11, dans laquelle la concentration en hyaluronane ou en ses dérivés est comprise entre 0,02 et 50 mg/ml.

14. Utilisation selon la revendication 13, dans laquelle la concentration en hyaluronane ou en ses dérivés est comprise entre 2 et 20 mg/ml.

15. Utilisation selon la revendication 14, dans laquelle la composition est administrée par voie intramusculaire.

16. Utilisation selon la revendication 11, dans laquelle la composition contient en outre du collagène, de la gélatine, un facteur de croissance ou une combinaison de ceux-ci.

17. Utilisation selon la revendication 11, dans laquelle la composition est exempte de facteur de croissance et de cellule.

18. Utilisation selon la revendication 17, dans laquelle la composition contient en outre du collagène.

19. Utilisation selon l'une quelconque des revendications 11 à 18, dans laquelle la composition est administrée à un être humain.
